# EUROPEAN PATENT APPLICATION

(11) **EP 4 729 882 A1**
(43) Date of publication of application: **22.04.2026**
(21) Application number: 24206948.2
(22) Date of filing: 16.10.2024
(51) Int. Cl.: G01B 11/16, A61B 5/06, G01L 1/24, G02B 6/02

(54) **MULTICORE OPTICAL FIBER AND ELONGATED DEVICE FOR MEDICAL INTERVENTIONAL APPLICATION**

(71) Applicant: Koninklijke Philips N.V., 5656 AG Eindhoven (NL)
(72) Inventor: MARCELIS, Bout, Eindhoven (NL); SCHLEIPEN, Johannes Joseph Hubertina Barbara, Eindhoven (NL); CLABBERS, Tom Johannes Hubertus, 5656AG Eindhoven (NL); VAN DEN ENDE, Daan Anton, Eindhoven (NL)
(74) Representative: Philips Intellectual Property & Standards

(57) **Abstract**

The present invention relates to a multicore optical fiber (10) configured for shape sensing. The optical fiber (10) comprises an elongated main section (40) and a distal tip section (42) connected to the main section (40). The main section (40) comprises a first cladding (12a) and a plurality of first fiber cores (14a, 16a, 18a, 20a) embedded in the first cladding (12a), and the distal tip section (42) comprises a second cladding (12b) and a plurality of second fiber cores (14b, 16b, 18b, 20b) embedded in the second cladding (12b). The first and second fiber cores (14a, 14b, 16a, 16b, 18a, 18b, 20a, 20b) comprise reflective strain sensitive structures (21) configured for distributed shape sensing along the main section (40) and the distal tip section (42). The main section (40) comprises a first material and the distal tip section (42) comprises a second material different from the first material, wherein a first strain at break of the main section (40) is lower than a second strain at break of the distal tip section (42).

## Description

### FIELD OF THE INVENTION

The present invention generally relates to the field of optical shape sensing technology, also referred to as Fiber Optic RealShape (FORS) technology. In particular, the present invention relates to a multicore optical fiber and an elongated device for medical interventional application comprising a multicore optical fiber.

### BACKGROUND OF THE INVENTION

Fiber Optic RealShape (FORS) technology is a shape sensing and reconstruction technology used in elongated devices, like guidewires, to enable accurate, three-dimensional and real-time visualization of the devices to allow efficient navigation during minimally invasive endovascular procedures without the need for X-ray guidance. The reduction of X-ray radiation exposure during such a procedure is beneficial for both patients as well as physicians and staff.

The FORS technology uses a shape sensing enabled elongated device, like a guidewire, for use in the procedure and a measurement system, which typically comprises a light source and an optical detector compatible with the shape sensing enabled elongated device, as well as a signal processing unit and a user interface for viewing and tracking the visualized elongated device.

FORS-enabled devices typically include an integrated twisted multicore optical glass fiber with sensors which may be configured as distributed fiber Bragg grating (FBG) sensors. Light from the light source is used to interrogate the sensors and reflections are analyzed to determine the local deformation of each segment of the optical fiber such that the full three-dimensional shape can be reconstructed. In this way, the full shape of elongated devices can be reconstructed and visualized in real time during the procedure. The FORS enabled devices may be visualized in the context of the patient's anatomy through overlay on images acquired before or during the procedure, like CT scans and X-rays. The amount of images are thus significantly reduced compared to X-ray guided procedures where a new X-ray image is needed every time the elongated device needs to be visualized, leading to a marked reduction in X-ray dose.

The applicant of the present application has developed FORS enabled guidewires and catheters, which have been successfully employed in complex aortic interventional procedures, in particular fenestrated endovascular aortic aneurysm repair (FEVAR) and/or branched endovascular aortic aneurysm repair (BEVAR), as well as in endovascular peripheral lesion repair (EVPLR).

In complex aortic procedures the requirements for mechanical properties of the tip section of the typically used guidewires do not restrict the use of brittle glass fiber sensors which extend up to the distal tip of the elongated device, since navigation often is within large vessels (e.g. aorta), where the guidewire is free to navigate inside the vessel and will typically not encounter obstructions.

In other procedures, such as various peripheral procedures, such as treatment of peripheral artery disease (PAD), FORS enabled elongated devices are also beneficial for reducing radiation dose. However, in these types of procedures, vessels are much smaller in diameter, and often the trajectory of navigation is very tortuous, i.e. with many bends, and potentially many bifurcations must be accessed to get to the target location, often involving acute angles through which the elongated device must travel. Moreover, these smaller vessels are much more fragile and may be damaged by the tip of the elongated device. Especially in PAD, angioplasty techniques require crossing a narrowed or occluded section of a blood vessel. For more severe occlusions, crossing a narrowed or occluded section of a blood vessel requires force, and sometimes the tip of the device prolapses, i.e. the device loops and the tip of the device bends back on itself, a phenomenon also called 'knuckling', causing very tight bending in the device tip, i.e. a bending radius < 1 mm. In these cases, the device tip must also be robust enough to withstand this deformation.

For FORS enabled devices, like guidewires, used in peripheral interventional procedures, the tip of the device must be soft and flexible enough to easily navigate through tight bends and acutely angled bifurcations. For example, the tip load of the combination of the inner optical fiber and the outer shell tip of the device must not exceed the limits required for safe navigation, i.e. without damaging the blood vessels, which is around 2-4 gram force for guidewires used in peripheral procedures.

Also during these procedures, the curvature of the optical fiber may become high enough to impede shape sensing or may even become higher than the maximum curvature that the glass fiber can withstand, resulting in failure of the glass material, i.e. breaking or shattering, during use.

In particular, during procedures where a tortuous anatomy must be navigated and/or a narrow lesion or occlusion must be crossed the forces acting on the tip of the device may result in deformations of the device tip which are too extreme for a glass fiber to handle, especially when the device tip is looped or prolapsed. Breaking of the glass fiber will not only impede the use of the FORS technology, but may also result in a patient safety issue if the glass splinters potentially damage the internal structure of the device tip, further altering its mechanical properties.

At the same time, the shape and direction of the tip of the elongated device must still be reconstructed by the FORS technology as accurate as possible even under such challenging circumstances, involving large deformations due to looping and prolapse, resulting in a tight bending radius, such that the need for X-ray guidance can still be avoided.

Polymer optical fibers are known which are more flexible than glass optical fibers, but they show increased optical losses compared to glass optical fibers.

### SUMMARY OF THE INVENTION

It is an object of the present invention to provide a multicore optical fiber configured for optical shape sensing, in particular for use in an elongated device for medical interventional application, which is suitable for procedures where a tortuous anatomy must be navigated and/or a narrow lesion or occlusion must be crossed, while at the same time allowing reconstruction of the shape and direction of the tip region of the optical fiber.

It is a further object to provide an elongated device for medical interventional application which is shape sensing enabled and suitable for procedures where a tortuous anatomy must be navigated and/or a narrow lesion or occlusion must be crossed, while at the same time allowing reconstruction of the shape and direction of the tip region of the elongated device by the FORS technology with sufficient accuracy.

In a first aspect of the present invention, a multicore optical fiber configured for shape sensing is provided, comprising an elongated main section and a distal tip section connected to the main section, the main section comprising a first cladding and a plurality of first fiber cores embedded in the first cladding, the distal tip section comprising a second cladding and a plurality of second fiber cores embedded in the second cladding, the first and second fiber cores comprising reflective strain sensitive structures configured for distributed shape sensing along the main section and the distal tip section, wherein the main section comprises a first material and the distal tip section comprises a second material different from the first material.

The present invention addresses the object underlying the invention by providing a multicore optical fiber which comprises a material in the distal tip section a material which is different from a material in the main section.

In a first embodiment, a first strain at break of the main section is lower than a second strain at break of the distal tip section. According to this first embodiment, a material in the distal tip section is different from a material in the main section such that the distal tip section has a higher strain at break than that of the main section. That is, in the distal tip section of the optical fiber where the bending radius is expected to be lower in use of the optical fiber, i.e. where the curvature is expected to be higher, the material of the main section is replaced by a material which is more mechanically robust such that the optical fiber can mechanically withstand higher curvatures without the risk of breaking. The material of the fiber cores and/or cladding of the distal tip section of the optical fiber may be different from the material of the fiber cores and/or cladding of the main section. The material of the main section where low bending radii or high curvatures are not to be expected during use may be chosen to provide high quality optical shape sensing. Further, the material of the main section may be selected for limited optical loss and low temperature sensitivity. The distal tip section may be optimized for high deformations, i.e. the material of the distal tip section may be chosen to provide higher mechanical robustness with respect to low bending radii or, in other words, high curvatures. Thus, the risk of breaking of the optical fiber in the distal tip section is reduced, while shape and direction of the distal tip section can still be accurately reconstructed by the FORS technology, such that the need for X-ray guidance is still avoided, even under challenging circumstances involving large deformations due to looping and prolapse of the distal tip region of an elongated device comprising the optical fiber.

The reflective strain sensitive structures may comprise fiber Bragg gratings (FBGs).

Other preferred embodiments of the invention are defined in the dependent claims and/or are described herein.

In a more particular embodiment, the second strain and break is larger than the first strain at break by a factor of at least 2, preferably at least 5, further preferably by a factor of at least 10, or by a factor of about 10.

The higher the strain at break of the distal tip section, i.e. the more elastic the distal tip section is, the more mechanically robust the distal tip section is to withstand high curvatures. For example, the material of the distal tip section of the optical fiber may be chosen to withstand bending radii of less than 2 mm, preferably less than 1 mm.

In a second embodiment, considered alone or in combination with said first embodiment, the distal tip section may have a bending strain sensitivity that is lower than that of the main section such that a first minimum bending radius of the distal tip section which is measurable by optically interrogating the optical fiber in a defined scan wavelength range is smaller than a second minimum bending radius of the main section which is measurable by optically interrogating the optical fiber in said defined scan wavelength range.

In optical shape sensing, an optical fiber is typically optically interrogated by sending light into the optical fiber, wherein the wavelength of the light is scanned over a certain wavelength range. For example, the wavelength range may be centered around 1550 nm with a scan range of 30 nm (i.e. the scan wavelength range is from 1535 nm to 1565 nm, for example). If the curvature of the optical fiber exceeds a certain curvature value, the wavelength shift of the reflected light due to strain may fall out of the scan range so that such a high curvature cannot be optically sensed anymore and shape reconstruction fails. That is, there is a minimum bending radius of the optical fiber which can still be measured by optical shape sensing, while bending radii below this minimum bending radius are not measurable. Since the distal tip section when used in an elongated device when navigated through tight bends may be subject to very low bending radii, it is thus advantageous if the bending strain sensitivity of the distal tip section is lower in comparison to the bending strain sensitivity of the main section of the optical fiber, so that minimum bending radii of the distal tip section may still be measured by the optical interrogator system, which bending radii are lower or much lower than the minimum bending radii which can be measured in the main section of the optical fiber.

In a third embodiment, considered alone or in combination with said first and/or second embodiment(s), the first fiber cores comprise outer cores arranged at the first radial distance from a center axis of the main section, and the second fiber cores comprise second outer cores arranged at a second radial distance from the center axis of the distal tip section, wherein the second radial distance is smaller than the first radial distance.

When the fiber core radial distance from the center axis is reduced, the bending strain sensitivity decreases with the advantage that the distal tip section not only is mechanically more robust to withstand high curvatures, but also allows to measure such high curvatures.

In a fourth embodiment, the first material may be silica glass.

In this embodiment, the main section may be made in total or at least in the major part along its length of silica glass. Silica glass has been proven to provide high quality shape sensing, with properties such as limited optical loss and low temperature sensitivity, especially if the reflective strain sensitive structures are fiber Bragg gratings. In this embodiment, the fiber cores and the cladding of the main section of the optical fiber may be made of silica glass.

In a further embodiment, the second material may be a polymer.

Polymers are known for their higher elasticity in comparison with silica glass and thus exhibit a much higher strain and break than silica glass. The distal tip section comprising a polymer can bend to a lower radius before failing. In this embodiment, at least the cladding of the distal tip section may be made of a polymer, while the fiber cores of the distal tip section may be made of the same polymer or a material different from a polymer. The higher optical losses of polymer fibers in comparison with silica fibers will not have much impact on the shape sensing performance of the distal tip section when the length of the distal tip section is small. A short distal tip section is sufficient for use in a highly flexible tip region of an elongated device which is subject to high curvatures during navigation.

In the context with the previous embodiment, the optical fiber may comprise a combination of silica glass in the main section and a polymer in the distal tip section.

The distal tip section comprising a polymer may be connected to the silica glass main section by any one of fusing, splicing, glueing, for instance using an ultraviolet (UV)-curable adhesive around the interface between the distal tip section and the main section after aligning the fiber cores of the distal tip section with the fiber cores of the main section.

The polymer comprised by the distal tip section may comprise one or more of PMMA, polystyrene, polycarbonate, TOPAS polymer, CEONEX polymer, CYTOP polymer.

TOPAS, CEONEX and CYTOP are special optical fiber polymers which exhibit a lower optical loss at higher wavelengths than PMMA while retaining mechanical robustness.

In a further embodiment, the second fiber cores may be made of silica glass.

Thus, within the scope of the present invention, the silica glass main section may be combined with a polymer distal tip section, wherein only the cladding of the distal tip section is made of a polymer, while the fiber cores of the distal tip section are made of silica glass. Thus, the distal tip section may be a silica glass/polymer hybrid. The polymer cladding provides for the mechanical robustness, while the silica glass fiber cores may provide for lower optical loss in comparison with fiber cores made of a polymer and improved shape sensing performance.

The lower mechanical robustness of silica glass fiber cores in comparison with polymer fiber cores may be at least in part compensated in combination with embodiment described above, according to which the fiber cores of the distal tip section have a reduced radial distance from the center axis of the distal tip section.

When manufacturing the distal tip section, a polymer cladding with holes may be produced in which the silica glass fiber cores may be inserted, optionally followed by an annealing step to remove the air gaps between the fiber cores and the cladding. An advantage is that due to the lower polymer melting or softening temperature, the FBGs may be written into the fiber cores before embedding them in the polymer cladding without the risk that the FBGs fade during subsequent processing.

In a further embodiment, considered alone or in combination with any of said aforementioned embodiments, the fiber cores of the distal tip section may be made of a first polymer and the cladding of the distal tip section may comprise a second polymer different from the first polymer, wherein the second polymer has an elastic modulus lower than that of the first polymer.

In this embodiment, the fiber cores of the distal tip section as well as the cladding of the distal tip section may be made of a polymer, wherein the polymer of the cladding of the distal tip section differs from the polymer of the fiber cores of the distal tip section. Especially, the polymer of the cladding of the distal tip section may be more elastic than the polymer of the fiber cores, expressed by a lower elastic modulus of the cladding of the distal tip section.

As an advantage of this embodiment, the manufacturing complexity of the distal tip section may be reduced, or at least allows for different manufacturing processes, because the elastomer cladding material is processable as a liquid at room temperature, or at least far below the temperature at which FBGs will fade, such that FBGs can be written before further processing the elastomer cladding. This also allows the fiber cores to be positioned as desired before the liquid cladding is back filled over the fiber core structure, allowing tapering and other structural changes over the length of the distal tip section, for instance a lower radial fiber core distance from the center axis or a twist profile of the fiber cores.

In the context of the previous embodiment, the fiber cores may be made of a conventional polymer optical fiber material that is FBG compatible (e.g. PMMA, TOPAS, CYTOP or PC, among others), while the cladding may be made of an elastomer, for instance such as thermosetting elastomer material like polydimethylsiloxane or polyurethane methacrylate. A UV-curable polymer for the cladding is possible as well.

In a further embodiment, the second material the distal tip section comprises, may be a polymer modified to lower the strain sensitivity of the polymer.

Typically, fiber cores comprising FBGs, which are made of a polymer, often have a higher strain sensitivity than silica glass optical fibers due to a lower strain-optic coefficient. The present embodiment has the advantage that a lower bending strain sensitivity of the distal tip section can be achieved to alleviate the higher strain sensitivity of polymers. Modifications of the polymer of the distal tip section may comprise removal of materials of, e.g. by etching, the distal tip section, for instance etching PMMA in acetone, a shorter inscription time of the reflective strain sensitive structures, or annealing, which is a combination of temperature and humidity treatment. This tip may alternatively or in combination be mechanically flattened.

In this way, the strain sensitivity of the highly flexible polymer distal tip section can be tuned to match the maximum measurable bending strain, such that the minimum bending radius of the local curvature main section matches the lower measurable minimum bending radius of the high curvature distal tip section.

In all embodiments above, the distal tip section may be spliced, glued or fused to the main section.

In a further embodiment, the distal tip section may have a length in a range from 1 to 5 cm.

In a further embodiment, the width or diameter of the tip may have been reduced, by e.g. material removal e.g. by etching, and/or mechanical flattening.

In a second aspect of the present invention, an elongated device for medical interventional application is provided, comprising:
a main section and a distal terminal section together defining a lumen along a length of the elongated device; and
a multicore optical fiber according to the first aspect which is arranged in the lumen, wherein the distal tip section of the optical fiber is arranged in the distal terminal section of the elongated device.

The elongated device comprising the multicore optical fiber according to the invention is particularly suitable for procedures where a tortuous anatomy must be navigated and/or a narrow lesion or occlusion must be crossed, due to the advantageous properties of the optical fiber, particularly the enhanced elasticity and mechanical robustness of the distal tip section of the optical fiber according to the invention.

In an embodiment of the elongated device, the distal terminal section may have a bending stiffness lower than that of the main section.

In particular, the distal terminal section of the elongated device may comprise a coil which is advantageously soft for interventional applications where a tortuous anatomy must be followed by the elongated device due to the high flexibility of the coil.

### BRIEF DESCRIPTION OF THE DRAWINGS

These and other aspects of the invention will be apparent from and elucidated with reference to the embodiments described hereinafter. In the following drawings:
Fig. 1 shows a piece of length of an example of a multicore twisted optical fiber;
Fig. 2 shows an embodiment of a distal region of an elongated device for medical interventional application with a distal region of an embodiment of an optical fiber arranged in the elongated device;
Fig. 3 shows a diagram depicting optical losses of polymer optical fibers and a silica glass fiber;
Fig. 4 shows a longitudinal section of a distal region of an optical fiber according to another embodiment; and
Fig. 5A and Fig. 5B show cross sections of a of an optical fiber with different fiber core radial distances from a center axis of the optical fiber.

### DETAILED DESCRIPTION OF THE INVENTION

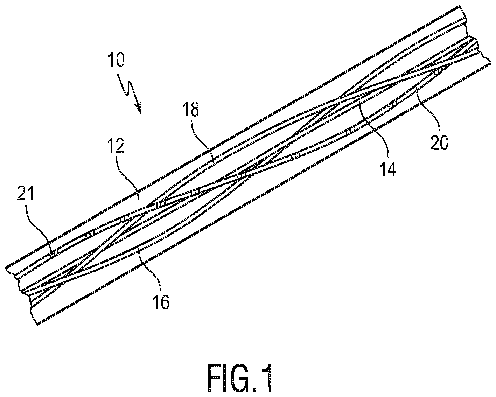

Fig. 1 shows a piece of a multicore optical fiber 10 configured for distributed shape sensing along the optical fiber 10. The multicore optical fiber 10 comprises a cladding 12 and a plurality of fiber cores 14, 16, 18, 20. The fiber core 14 is a central core arranged on and along the center axis of the optical fiber 10. Fiber cores 16, 18, 20 are outer fiber cores arranged radially apart from the center core 14. The outer cores 16, 18, 20 may be helically wound around the central fiber core 14 as shown. The outer cores 16, 18, 20 are angularly spaced with respect to one another around the longitudinal center axis of the optical fiber 10. The angular spacing between neighboring fiber cores typically is 120° for a number of three outer fiber cores. The multicore optical fiber 10 may have less than four fiber cores, or more than four fiber cores. For example, the multiple optical fiber 10 may comprise seven fiber cores, with three radially outer fiber cores, one central fiber core, and three other fiber cores in an intermediate radial position between the three outer cores and the central core.

Each of the fiber cores 14, 16, 18, 20 comprises a reflective strain sensitive structure 21 exemplarily shown for fiber core 20 in Fig. 1. The reflective strain sensitive structure may comprise one or more fiber Bragg gratings (FBGs). The reflective strain sensitive structures 21, e.g. the FBG or FBGs, are responsive to strain in the optical fiber 10. The multicore optical fiber 10 can sense strain, which may be axial strain, bending strain or torsional strain, such that 3D shape reconstruction of the optical fiber 10 can be performed with an optical interrogator system (not shown) as known in the art. When the multicore optical fiber 10 is used in an elongated device, like a guidewire or catheter, it is thus possible to provide a 3D shape reconstruction of the elongated device.

The optical interrogator system sends laser light into the fiber cores 14, 16, 18, 20 of the optical fiber 10, and then receives and analyzes the returning light reflected from the reflective strain sensitive structures 21. The wavelength of the laser light sent through the optical fiber typically is scanned over a range of wavelengths, e.g. in a range from 1535 nm to 1565 nm. Twists and bends in the optical fiber influence the wavelength spectrum of the light returned into the optical interrogator system. By analyzing the wavelength spectra of the returned light from the fiber cores 14, 16, 18, 20, it is possible to reconstruct the 3D shape of the full length of the optical fiber 10 and thus of an elongated device in which the optical fiber is arranged. Thus, radiation-free visualization of an elongated device, for example a guidewire introduced into the vasculature of a patient, in real time and in 3D is enabled.

Typical multicore optical fibers made of silica glass with an outer cladding diameter in a range of e.g. 100 µm - 125 µm have been proven to provide good strain sensitivity and, therefore, are very suitable for the FORS technology. In medical interventional applications, such as peripheral procedures, e.g. treatment of peripheral artery disease, fiber optical real shape (FORS) enabled elongated devices equipped with a multicore optical fiber, especially FORS enabled guidewires, are beneficial for reducing radiation dose. However, in these types of procedures, vessels are very small in diameter, and often the trajectory of navigation is very tortuous (i.e. with many bends), and potentially many bifurcations must be accessed to get to the target location, often involving acute angles through which the elongated device and thus the multicore optical fiber must travel. Moreover, these smaller vessels are much more fragile and may be damaged by the tip of the elongated device. Especially in PAD, angioplasty techniques require crossing a narrowed or occluded section in a blood vessel. For more severe occlusions, this typically requires force and sometimes the tip of the wire prolapses, i.e. the wire loops and the tip bends back on itself, a phenomenon also called 'knuckling', which causes very tight bending in the tip of the device, e.g. with a bending radius < 1 mm.

Wang, Jian-Bo et al. "An Effective Guidewire Looping Technique for the Recanalization of Occlusive Segments of Infrapopliteal Vessels", Korean Journal of Radiology 11 (2010), pages 441 - 448, describe such a transluminal angioplasty technique. As described there, a hydrophilic guidewire is inserted into a proximal branch of the vessel and then a "U" shape is made by rotating and advancing the guidewire continuously. The looped guidewire is then advanced into and over occlusive segments of the vessel, whereafter a catheter is crossed over the occlusive segment along the guidewire. The guidewire described there is not equipped with an optical fiber.

When the elongated device, like a guidewire, includes an optical fiber (like optical fiber 10 in Fig. 1) made of silica glass and extending up to the distal tip of the elongated device, and when the optical fiber is subject to a loop formation as described above, the curvature of the optical fiber may become too high so that shape sensing is impeded, or, when the curvature of the optical fiber becomes even higher than the maximum curvature that the glass fiber can withstand, this results in failure of the glass material of the optical fiber, i.e. the optical fiber may break or shatter.

These technical problems are solved by a new design of a multicore optical fiber, embodiments of which will be described below. The present invention addresses the technical problems described above by replacing the material of a section, in particular the distal tip section, of the optical fiber 10 in areas, where the bending radius is expected to be lower, or in other words the curvature is expected to be higher, with a material that is better suited to the higher curvature, at least in terms of mechanical robustness, and optionally also in terms of strain sensing ability. By selecting a more mechanically robust material, the optical fiber can mechanically survive the higher curvature. In some embodiments, the distal tip section of the optical fiber may be additionally configured such that its sensitivity to bending strain is reduced such that the maximum curvature which can be sensed by the distal tip section, when optically interrogating the optical fiber, is increased.

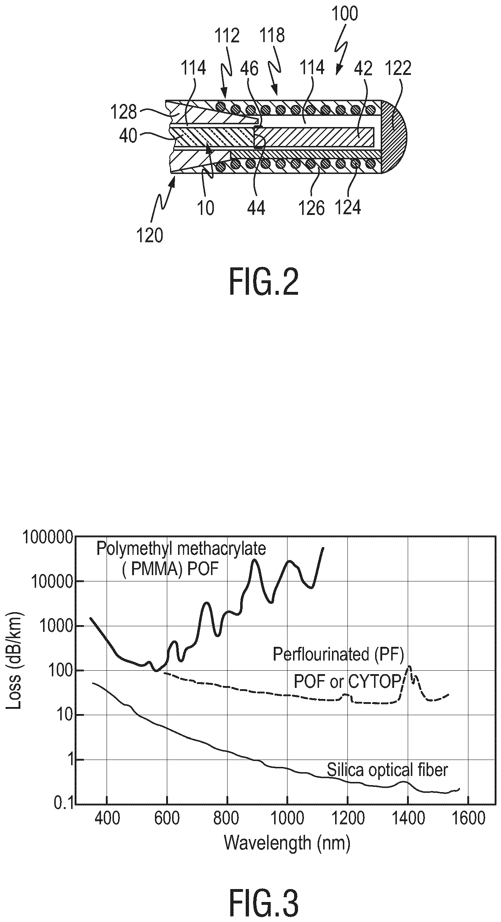

Fig. 2 shows a distal region of an elongated device 100 for medical interventional application. The elongated device 100 comprises a shell 112 defining a lumen 114 extending in longitudinal direction along the length of the device 100. An optical fiber 10, in particular a multicore optical fiber 10, is arranged in the lumen 114. The optical fiber 10 may be free to slide and rotate in the lumen 114. The optical fiber 10 thus may be a free floating optical fiber in the lumen 114.

The device 100 comprises a distal terminal section 118 and a main section 120 arranged proximally of the distal terminal section 118. The distal terminal section 118 of the device 100 terminates at a distal tip 122. The distal terminal section 118 may comprise a coil 124 embedded in a coating 126, for example a polyurethane coating. The main section 120 of the device 100 may comprise a tube 128, e.g. a nitinol hypotube, or a tube which may have cuts in its periphery.

The distal terminal section 118 of the device 100 may have a bending stiffness which is lower, preferably much lower than the bending stiffness of the main section 120 of the device 100. In other words, the distal terminal section 118 is softer/more flexible than the main section 120 such that the distal terminal section 118 may prolapse when the elongated device 100 is advanced in a blood vessel and encounters an occlusion or lesion. In the following, the optical fiber 10 will be described in more detail.

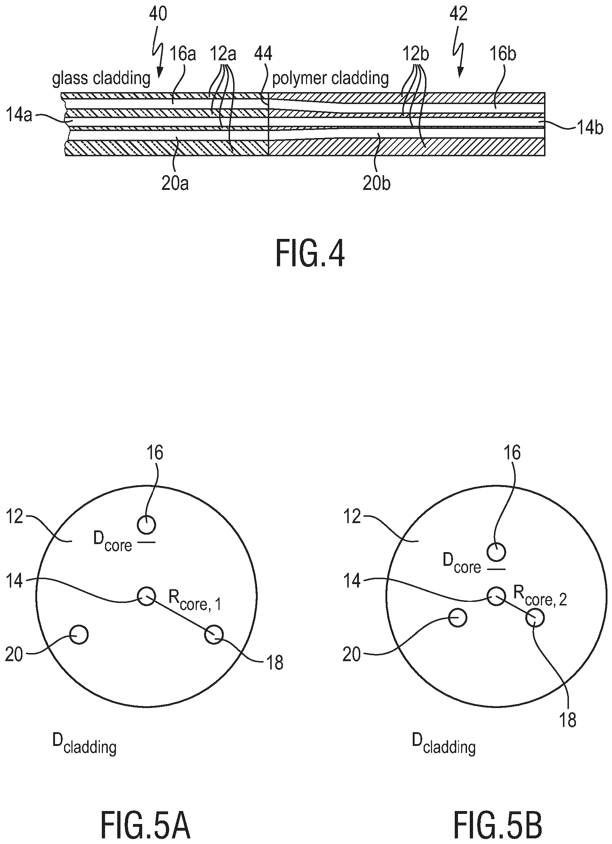

The optical fiber 10 comprises a main section 40 and a distal tip section 42. The distal tip section 42 is a section of the optical fiber which may be exposed to high bending curvatures, or synonymously, to low bending radii. The main section 40 and the distal tip section 42 each may comprise a cladding commonly labeled with reference numeral 12 and fiber cores commonly labeled with reference numerals 14, 16, 18, 20 as described above with reference to Fig. 1. As for example shown in Fig. 4, the cladding of the main section 40 is also labeled with reference numeral 12a, and the fiber cores of the main section 40 with reference numerals 14a, 16a, 18a, 20a. The cladding of the distal tip section 42 is also labeled with reference numeral 12b, and the fiber cores of the distal tip section 42 with reference numerals 14b, 16b, 18b, 20b A lower or higher number of fiber cores than 4 is possible, e.g. 2, 3, 5, 6, 7. The distal tip section 42 of the optical fiber 10 comprises or is made of a material which is different from a material of the main section 40. The material of the cladding 12b and/or the fiber cores 14b, 16b, 18b, 20b of the distal tip section 42 are selected such that the distal tip section 42 has an elasticity which is higher than an elasticity of the main section 40. In other words, the material or materials of the distal tip section 42 are chosen such that the distal tip section 42 is mechanically more robust than the main section 40. In particular, the material of the distal tip section 42 is selected such that the strain at break of the distal tip section 42 is higher than the strain at break of the main section 40. Preferably, the strain at break of the distal tip section 42 is larger than the first strain at break by a factor of at least 2, preferably at least 5, further preferably by a factor of at least 10.

In an embodiment, the main section 40 of the optical fiber 10 may be made of silica glass, i.e. the material of cladding 12a as well as the material of fiber cores 14a, 16a, 18a, 20a may be silica glass, while the distal tip section 42 may be entirely made of or comprise a polymer, in particular an optical polymer, which can have a much higher strain at break (by a factor of more than 10) than glass fibers and, therefore, can bend to a lower radius before failing. In this embodiment, the cladding 12b as well as the fiber cores 14b, 16b, 18b, 20b of the distal tip section 42 may be made of a same polymer.

The mechanically more robust polymer may be fused, spliced or glued to the main section 40 of the optical fiber 10. For instance, a UV-curable adhesive 46 may be used around a joint 44 between the distal tip section 42 and the main section 40 after aligning the fiber cores of the distal tip section 42 with the fiber cores of the main section 40.

It is known that polymers, e.g. optical PMMA, show high optical loss per unit length around a wavelength of 1550 nm such that it is not advantageous to fabricate the full length optical fiber 10 including the main section 40 out of this material. However, the distal terminal section 118 of an elongated device, like a guidewire, is typically short, for instance 2-4 cm, so that even with the higher optical loss, the short length of the distal tip section 42, which may be in a range from 1 to 5 cm, preferably 2 to 4 cm, arranged in the distal terminal section 118 of the elongated device 100 limits the absolute loss in signal to acceptable levels. PMMA, as most other polymers, exhibit much better mechanically robustness than silica glass. While the maximum mechanical strain limit of glass fibers is less than about 2 % (equivalent to 20,000 µε), polymers such as PMMA may be strained to much higher elongations before breaking, typically more than 10 times as high. Moreover, instead of brittle fracture of glass fibers, the polymer fibers will yield and although this will influence the sensing characteristics (causing any combination of hysteresis, increased noise or loss of signal) it may still be possible to reconstruct a shape of the optical fiber 10 up to the distal tip, albeit with less accuracy, whereas with a broken glass fiber, shape reconstruction will be impossible altogether. Up to the yield point (over 20,000 µε) the strain sensing characteristics of polymer fibers are linear or almost linear. Effectively these improved mechanical properties of polymers used for the distal tip section 42 of the optical fiber 10 allow for a much more mechanically robust distal tip section 42 of the optical fiber 10 that does not break during prolapsing or looping of the distal terminal section 118 of the device 100.

Other materials which may be used for the cladding 12b and/or the fiber cores 14b, 16b, 18b, 20b of the distal tip section 42 of the optical fiber 10 may include polystyrene (PS), polycarbonate (PC) and special optical fiber polymers, such as TOPAS, ZEONEX and CYTOP polymers. These special materials exhibit an improved optical loss at higher wavelengths than PMMA while retaining the high mechanical robustness. Fig. 3 shows the optical loss of polymer optical fibers for a PMMA optical fiber, a CYTOP optical fiber and a silica glass optical fiber in dependence on wavelength.

The strain sensitivity of polymer optical fibers is typically higher than that of silica glass optical fibers, both when equipped with fiber Bragg gratings, by around 20-40 % (i.e. 1.4-1.7 pm/µε of polymer fibers compared to 1.2 pm/µε for glass fibers), due to the improved strain-optic coefficient of optical polymers.

In case of a single optical interrogator system that senses both the main section 40 made of glass as well as the distal tip section 42 made of polymer with a fixed wavelength scan range, it would be more advantageous to have a reduced bending strain sensitivity of the distal tip section 42 that is subject to high curvature bending, i.e. a lower wavelength shift per unit of curvature in the distal tip section 42 would be advantageous. Although counterintuitive, the advantage of having less sensitivity to curvature in the distal tip section 42 of the optical fiber is that the wavelength scan range of the interrogation light source can stay the same, but the wavelength shift caused by the larger maximum curvature of the high curvature distal tip section 42 still falls within this scan range. Thus, the minimum bending radius of the distal tip section 42 which can be measured by optically interrogating the optical fiber 10 in a defined scan wavelength range is smaller than the minimum bending radius of the main section which can be measured by optically interrogating the optical fiber 10 in said defined scan wavelength range.

To reduce the bending strain sensitivity of the distal tip section 42, a further embodiment schematically shown in Fig. 4 provides that the radial distance of the outer optical cores (outer fiber cores 16b and 20b are shown in Fig. 4, without helicity for the sake of simplicity of the drawing) from the neutral line or central core 14b is variable, for instance decreasing distally as shown in Fig. 4. When the outer fiber core radial distance, i.e. the radial distance of the outer cores from the neutral line of the fiber 10, r_{core}, decreases, the strain sensitivity to bending decreases, i.e. the same bending curvature induces less strain in the outer fiber cores by the ratio of the fiber core distances, *R*_{*core*,2}/*R*_{*core*,1}, where *R*_{*core*,2} *< R*_{*core*,1}, see Figs 5A and 5B. This is because the average strain due to bending in the fiber core follows the relation *ε_{core} = r_{core}*/*R_{bend},* with *R_{bend}* being the bending radius

In terms of manufacturing the distal tip section 42 with decreasing radial distances of the outer fiber cores 16b, 18b, 20b as shown in Fig. 4 it is to be noted that polymer optical fibers can be stretched more easily during processing than silica (glass) fibers due to their flexibility and lower melting temperature to achieve this reduction in core radial distance so that this method can be more easily applied to polymer fibers than to glass fibers. At the interface 44, the outer fiber core radial distances in the distal tip section 42 must match the outer fiber core radial distance in the main section 40 as shown in Fig. 4.

Another advantage of the reduced strain per unit of curvature in the outer fiber cores in the distal tip section 42 due to the reduced radial distance of the outer fiber cores is that birefringence effects are limited, which can be beneficial for accuracy of the strain sensing and corresponding shape calculations.

In an alternative embodiment, the optical fiber 10 may be designed such that the fiber cores 14b, 16b, 18b, 20b of the distal tip section 42 are made of glass material, which preserves the low optical loss characteristics, while the cladding 12b around the cores 14b, 16b, 18b, 20b is made of polymer material, which is more elastic and thus mechanically more robust or tougher than glass. Although not as mechanical robust as the distal tip section 42 design in the embodiment described with reference to Fig. 2 above because of the glass material in the fiber cores 14b, 16b, 18b, 20b, the fiber cores 14b, 16b, 18b, 20b are typically not located on the outer diameter of the optical fiber 10 so that the bending strain in the fiber cores is slightly lower than in the outer region of the cladding 12b.

For a typical fiber geometry with a diameter D_{cladding} (see Figs 5A and 5B) of 100 µm and a core diameter D_{core} of 8 µm with the outer core radial distance being around 35 µm, the maximum strain in the cladding 12 is equal to *D_{cladding}*/2*R_{bend}* = 50 µ*m*/*R_{bend}*, but the maximum strain in the outer fiber cores is (*D_{core}*/2 + *R_{core}* )/*R_{bend}* = (4 µ*m* + 35 µ*m*)/*R_{bend},* i.e. a reduction of about 25 %, which will allow lower bend radii before the glass of the fiber cores breaks.

For relatively short sections of the optical fiber 10 like distal tip section 42, an optical fiber can be manufactured by manufacturing a polymer cladding 12b with holes in which the glass fiber cores 14b, 16b, 18b, 20b may be inserted, optionally followed by an annealing step to remove air gaps between the fiber cores 14b, 16b, 18b, 20b and the cladding 12b. Manufacturing polymer optical fibers with holes is a known technique to produce micro-structured polymer optical fibers. Therefore, this embodiment is an alternative, when the application requires a low loss signal but still requires a higher mechanically robustness (i.e. a lower bending radius) than that of conventional glass fibers. It should be noted that this only holds for pure bending deformation, not for axial strain. In an axial strain situation, the strain on each fiber core is the same, regardless of the radial distance from the center.

Another advantage of this embodiment in which the fiber cores 14b, 16b, 18b, 20b are made of silica glass, while the cladding 12b is made of a polymer, is, due to the lower polymer melting or softening temperature, that reflective strain sensitive structures, e.g. FBGs, may be written into the fiber cores 14b, 16b, 18b, 20b, before embedding the fiber cores in the polymer cladding 12b without the risk that the FBGs fade during subsequent processing. For example, most polymers are processable, or at least deformable, at temperatures around 100 °C, a temperature at which FBGs in silica glass are stable. This allows adding or adjusting a twist ratio of the outer fiber cores as well as deformation of the optical fiber 10, such as adding a curvature angle without straining the cores.

Additionally, the outer core radial distance of the high curvature distal tip section 42 may be reduced compared to the low curvature main section 40 as described above with reference to Fig. 4. In this case, in addition to the advantage of having a locally reduced bending sensitivity, the strain induced in the outer fiber cores during bending is further reduced by a factor of *R*_{*core,*2}/*R*_{*core,*1} (see Figs 5A and 5B), and therefore the radius at break of the fiber cores 14, 16, 18, 20 also lowers further.

While in the embodiment described above the fiber cores 14b, 16b, 18b, 20b are silica (glass) fiber cores in the distal tip section 42, another embodiment provides that the fiber cores 14b, 16b, 18b, 20b are made of a first polymer, in particular a conventional polymer optical fiber material that is FBG compatible, e.g. PMMA, TOPAS, CYTOP or PC, amongst others, while the material of the cladding 12b comprises a second polymer, in particular an elastomer, having a lower elastic modulus than that of the first polymer the fiber cores 14b, 16b, 18b, 20b comprise. For example, the cladding 12b can comprise a thermosetting elastomer material like polydimethylsiloxane (PDMS) or polyurethane methacrylate (PUMA), or even UV curable polymers.

As another added benefit, this material choice reduces the manufacturing complexity, or at the very least allows for different manufacturing processes, since the elastomer material of the cladding 12b is processable as a liquid at room temperature, or at least far below the temperature at which the FBGs will fade, such that the FBGs can be written before further processing of the elastomer cladding 12. This also allows the fiber cores 14b, 16b, 18b, 20b to be positioned as desired before the liquid cladding material is backfilled over the fiber core structure, allowing tapering (see Fig. 4) and other structural changes over the length of the distal tip section 42, for instance a variable radial distance as shown in Fig. 4 or twist profile of the fiber cores 14b, 16b, 18b, 20b. Since the distal tip section 42 is typically much smaller in length than the main section 40, this type of processing can be feasible.

As described above, shape sensing enabled optical fibers, like those comprising FBGs, made of polymer often have a higher strain sensitivity than silica (glass) fibers due to a lower strain-optic coefficient, which higher strain sensitivity may offset the effect of reducing the radial distance of the outer cores as described above with reference to Figs 4 and 5B, which may be needed to avoid a wavelength shift of the FBGs due to bending that exceeds the wavelength scan range of the interrogator.

However, it is also possible to provide the distal tip section 42 with a polymer material that is less sensitive to strain than typical silica (glass) fibers. For example, it is possible that an optical fiber made of PMMA may show a strain sensitivity of less than 1 pm/µε, and CYTOP optical fibers may have a strain sensitivity of 1.1 pm/µε.

The intrinsic strain sensitivity of polymer optical fibers can be influenced by several factors, such that the intrinsic sensitivity to strain is reduced. Thus, in another embodiment, the distal tip section 42 comprising a polymer as the material for the cladding 12b and/or the fiber cores 14b, 16b, 18b, 20b may be modified, wherein the modification may include etching the polymer distal tip section 42 (for instance PMMA etched in acetone); a shorter inscription time for inscribing the FBGs into the fiber cores; and/or annealing, i.e. a combination of temperature and humidity environment.

In this way, the strain sensitivity of the highly flexible polymer distal tip section 42 of the optical fiber 10 can be tuned to match the maximum measurable bending strain, such that the minimum bending radius of the low curvature main section 40 of the optical fiber 10 matches the even lower minimum bending radius of the high curvature distal tip section 42.

Using the above technologies to reduce the strain sensitivity of the distal tip section 42, preferably in combination with the reduction of the radial outer core distances from the central core of the optical fiber 10 as described above, a 60-70 % reduction of the measurable minimum bending radius of the distal tip section 42 can be achieved, for instance from 2.5 mm for a glass fiber to less than 1 mm for a polymer optical fiber.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Multicore optical fiber configured for shape sensing, comprising an elongated main section (40) and a distal tip section (42) connected to the main section (40), the main section (40) comprising a first cladding (12a) and a plurality of first fiber cores (14a, 16a, 18a, 20a) embedded in the first cladding (12a), the distal tip section (42) comprising a second cladding (12b) and a plurality of second fiber cores (14b, 16b, 18b, 20b) embedded in the second cladding (12b), the first and second fiber cores (14a, 14b, 16a, 16b, 18a, 18b, 20a, 20b) comprising reflective strain sensitive structures (21) configured for distributed shape sensing along the main section (40) and the distal tip section (42), wherein the main section (40) comprises a first material and the distal tip section (42) comprises a second material different from the first material, wherein a first strain at break of the main section (40) is lower than a second strain at break of the distal tip section (42).

2. Multicore optical fiber of claim 1, wherein the second strain at break is larger than the first strain at break by a factor of at least 2, preferably at least 5, further preferably by a factor of at least 10.

3. Multicore optical fiber of claim 1 or 2, wherein the distal tip section (42) has a bending strain sensitivity that is lower than that of the main section (40) such that a first minimum bending radius of the distal tip section (42) which is measurable by optically interrogating the optical fiber (10) in a defined scan wavelength range is smaller than a second minimum bending radius of the main section (40) which is measurable by optically interrogating the optical fiber (10) in said defined scan wavelength range.

4. Multicore optical fiber of any one of claims 1 to 3, wherein the first fiber cores (14a, 16a, 18a, 20a) comprise outer cores (16a, 18a, 20a) arranged at a first radial distance from a center axis of the main section (40), and the second fiber cores (14b, 16b, 18b, 20b) comprise second outer cores (16b, 18b, 20b) arranged at a second radial distance from a center axis of the distal tip section (42), wherein the second radial distance is smaller than the first radial distance.

5. Multicore optical fiber of any one of claims 1 to 4, wherein the first material is silica glass.

6. Multicore optical fiber of any one of claims 1 to 5, wherein the second material is a polymer.

7. Multicore optical fiber of claim 6, wherein the polymer comprises one of PMMA, polystyrene, polycarbonate, TOPAS polymer, ZEONEX polymer, CYTOP polymer.

8. Multicore optical fiber of any one of claims 5 to 7, wherein the second fiber cores (14b, 16b, 18b, 20b) are made of silica glass.

9. Multicore optical fiber of any one of claims 1 to 4, wherein the second fiber cores (14b, 16b, 18b, 20b) are made of a first polymer and the second cladding (12b) comprises a second polymer different from the first polymer, wherein the second polymer has an elastic modulus lower than that of the first polymer.

10. Multicore optical fiber of claim 9, wherein the first polymer comprises one of PMMA, polystyrene, polycarbonate, TOPAS polymer, ZEONEX polymer, CYTOP polymer, and the second polymer comprises an elastomer, in particular one of a thermosetting elastomer, in particular polydimethylsiloxane, polyurethane methacrylate, or a polymer curable by ultraviolet light.

11. Multicore optical fiber of any one of claims 1 to 10, wherein the second material is a polymer modified to lower the strain sensitivity of the polymer.

12. Multicore optical fiber of any one of claims 1 to 11, wherein the distal tip section (42) is one of spliced, glued or fused to the main section.

13. Multicore optical fiber of any one of claims 1 to 12, wherein the distal tip section (42) has a length in a range from 1 to 5 cm.

14. Elongated device for medical interventional application, comprising:
a main section (120) and a distal terminal section (118) together defining a lumen (114) along a length of the elongated device (100); and
a multicore optical fiber (10) according to any one of claims 1 to 13 which is arranged in the lumen (114), wherein the distal tip section (42) of the optical fiber (10) is arranged in the distal terminal section (118) of the elongated device (100).

15. Elongated device of claim 14, wherein the distal terminal section (118) has a bending stiffness lower than that of the main section (120).
